(19) Europäisches Patentamt
European Patent Office
Office européen des brevets

(11) **EP 2 069 065 B1**

(12) **EUROPÄISCHE PATENTSCHRIFT**

(45) Veröffentlichungstag und Bekanntmachung des Hinweises auf die Patenterteilung:
**28.02.2018   Patentblatt 2018/09**

(21) Anmeldenummer: **07818706.9**

(22) Anmeldetag: **25.09.2007**

(51) Int Cl.:
**B01J 19/26** (2006.01)          **C07D 251/62** (2006.01)

(86) Internationale Anmeldenummer:
**PCT/EP2007/008628**

(87) Internationale Veröffentlichungsnummer:
**WO 2008/037500 (03.04.2008 Gazette 2008/14)**

(54) **VENTIL ZUM EINSPEISEN VON LÖSUNGEN IN KRISTALLISATIONSANLAGEN**

VALVE FOR FEEDING SOLUTIONS INTO CRYSTALLIZING PLANTS

SOUPAPE D'INTRODUCTION DE SOLUTIONS DANS DES DISPOSITIFS DE CRISTALLISATION

(84) Benannte Vertragsstaaten:
**AT BE BG CH CY CZ DE DK EE ES FI FR GB GR HU IE IS IT LI LT LU LV MC MT NL PL PT RO SE SI SK TR**

(30) Priorität: **26.09.2006   DE 102006047068**

(43) Veröffentlichungstag der Anmeldung:
**17.06.2009   Patentblatt 2009/25**

(73) Patentinhaber: **CASALE SA**
**6900 Lugano (CH)**

(72) Erfinder:
• **NEUMÜLLER, Christoph**
**4482 Ennsdorf (AT)**
• **EDER, Gerhard**
**4209 Engerwitzdorf (AT)**

(74) Vertreter: **Biazzi, Riccardo et al**
**M. ZARDI & CO. S.A.**
**Via Pioda, 6**
**6900 Lugano (CH)**

(56) Entgegenhaltungen:
EP-A- 1 566 382          WO-A-00/40566
WO-A-2004/002966          WO-A-2004/074265
US-A- 3 132 143

Bemerkungen:
Die Akte enthält technische Angaben, die nach dem Eingang der Anmeldung eingereicht wurden und die nicht in dieser Patentschrift enthalten sind.

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

EP 2 069 065 B1

## Beschreibung

Die Erfindung betrifft ein Ventil nach Anspruch 1, deren Verwendung nach Anspruch 4 eine Kristallisationsanlage nach Anspruch 5 und ein Verfahren nach Anspruch 6. Die Verwendung von Ventilen zur Mengenregelung beim Einspeisen oder Eindüsen von feststoffhaltigen Lösungen in einen Kristallisationskreislauf sind bekannt.

So wird z.B. zur Kristallisation von Melamin aus einer wässrigen Melaminlösung ein herkömmliches Druckventil verwendet, dessen Austrittsteil außerhalb der Strömung der Lösung des Kristallisationskreislaufes angeordnet ist. Bei dem herkömmlich verwendeten Ventil handelt es sich um ein Durchgangsventil.

Bei der herkömmlichen Melaminkristallisation wird eine wässrige erste Melaminlösung mit einem bestimmten Dampfdruck $p_{DTA}$ und einer bestimmten Temperatur $T_A$ in eine strömende zweite Lösung mit einem Gesamtdruck $p_{gesB}$, der sich aus dem Dampfdruck der Lösung und dem statischen Druck der Lösung ergibt, und einer niedrigeren Temperatur $T_B$ mittels eines Ventils eingespeist. Üblicherweise ist bei der herkömmlichen Melaminkristallisation der Gesamtdruck $p_{gesB}$ der zweiten Lösung geringer als der Dampfdruck $p_{DTA}$ der ersten Lösung ($p_{gesB} < p_{DTA}$).

Der Gesamtdruck der zweiten Lösung $p_{gesB}$ setzt sich aus Dampfdruck $p_{DTB}$ und statischem Druck $p_{statB}$ gemäß

$$p_{gesB} = p_{DTB} + p_{statB} \qquad (1)$$

zusammen.

Da der Gesamtdruck $p_{gesB}$ der zweiten Lösung geringer als der Dampfdruck $p_{DTA}$ der ersten Lösung ($p_{gesB} < p_{DTA}$), kommt es normalerweise am Einspeiseort der ersten Lösung in die zweite Lösung am Punkt des Druckabfalls zum so genannten "Flash-Effekt". Dieser Effekt wird durch eine schlagartige Entspannung der ersten Lösung am Punkt des Druckabfalles hervorgerufen.

Der "Flash-Effekt" ergibt sich aus der Differenz des Dampfdruckes der ersten Lösung und des Gesamtdruckes der zweiten Lösung an der Einspeisestelle und tritt dann auf, wenn der Dampfdruck der ersten Lösung größer ist als der Gesamtdruck der zweiten Lösung ($p_{DTA} > P_{gesB}$). Bei der Entspannung verdampft ein Teil der eingespeisten ersten Lösung unter Ausbildung von Gasblasen und es kommt zu einer Aufkonzentration der ersten Lösung.

Die herkömmliche Anordnung des Austrittsteils des Einspeiseventils außerhalb der Strömung des Kristallisationskreislaufes hat daher mehrere Nachteile.

Durch die Lage des Ventilaustrittteils außerhalb des Strömungskreislaufes der Kristallisationslösung kann es ebenfalls zu einer unerwünschten Auskristallisation des Feststoffes direkt im Ventil oder in dem kurzen Leitungsstück nach dem Ventil vor dem Einspeisepunkt in die Kristallisationslösung kommen. Im schlimmsten Fall hat dies ein Verstopfen des Ventils oder der Leitung zur Folge.

Des Weiteren sind die in der ersten melaminhaltigen Lösung entstehenden Gasblasen in der Regel sehr groß und unregelmäßig, d.h. die Größe und Anzahl der Gasblasen kann bei der Verwendung eines herkömmlichen Ventils nicht beeinflusst werden. Bei Kontakt der gebildeten Gasblasen mit dem kühleren Kreislaufstrom der zweiten Lösung erfolgt eine schlagartige Abkühlung der Gasblasen verbunden mit einer erneuten Kondensation. Dieser Effekt macht sich in Form von so genannten Dampfschlägen in der Kreislaufleitung bemerkbar, die eine sehr starke mechanische Beanspruchung der umliegenden Anlageteile, speziell der Pumpen und Armaturen, verursachen. So können die Leitungen mit Amplituden von mehreren Zentimetern schwingen.

Der Erfindung liegt nunmehr das Problem zu Grunde, ein Ventil zum Eindüsen einer feststoffhaltigen ersten Lösung oder Suspension in eine zweite Lösung zum Zwecke der Kristallisation bereitzustellen, welches ein frühzeitiges Auskristallisieren des Feststoffes und damit eine Verstopfung des Ventils verhindert und die mechanische Beanspruchung erheblich reduziert.

Diese Aufgabe wird durch die Bereitstellung eines Ventils mit den Merkmalen des Anspruchs 1 gelöst.

Das erfindungsgemäße Ventil weist dabei mindestens ein Austrittsteil zum Eindüsen einer feststoffhaltigen ersten Lösung in eine zweite Lösung zur kontinuierlichen Kristallisation des in der ersten Lösung gelösten Feststoffes in der zweiten Lösung. Das erfindungsgemäße Ventil ist dadurch gekennzeichnet, dass im Austrittsteil mindestens ein Ventilkegel angeordnet ist und der Ventilkegel aus einem Kegelstumpf, einem zylindrischen Teil und einer Kegelstange besteht.

Der Kegelstumpf erfüllt die Dichtfunktion in geschlossenem Zustand und der zylindrische Teil weist Austrittsöffnungen für den Durchtritt der ersten Lösung auf.

Das erfindungsgemäße Ventil ist somit derart gestaltet, dass der Druckabfall erst unmittelbar an der Stelle erfolgt, an der die Vermischung der ersten Lösung mit dem Kreislaufstrom der zweiten Lösung im Kristallisationsreaktor bzw. die Verdünnung mit der zweiten Lösung erfolgt.

Dadurch wird erreicht, dass der Druckabfall, d.h. der Punkt der Verdampfung der ersten Lösung und somit eine Konzentrationserhöhung desselbigen Mediums, an einer Stelle erfolgt, wo die Auskristallisation eines in der ersten Lösung gelösten Feststoffes, insbesondere Melamin, bereits erwünscht ist, d.h. im Kreislaufstrom des Kristallisationsreaktors. Das Auskristallisieren bewirkt an dieser Stelle keine Verstopfung von Leitungen oder Apparaten, da die gebildeten Kristalle sofort durch die zweite Lösung abgeführt werden.

Es ist dabei von Vorteil, wenn der Dampfdruck $p_{DTA}$ der ersten Lösung größer ist als der Gesamtdruck $p_{gesB}$ der zweiten Lösung.

Es ist weiterhin von Vorteil, wenn die erste Lösung einen gelösten Feststoff, insbesondere Melamin, mit einer Konzentration des Feststoffes in der Lösung $C_A$ und die zweite Lösung einen gelösten Feststoff, insbesondere Melamin, mit einer Konzentration des Feststoffes $C_B$ enthält, wobei $C_A$ größer ist als $C_B$.

Insbesondere ist es vorteilhaft, wenn das Austrittsteil des Ventils an seiner Kontaktfläche mit der zweiten Lösung, d.h. an der Ventilwand, eine Wärmeisolation aufweist. Die Wärmeisolation besteht bevorzugt aus einem Material mit schlechten Wärmeleiteigenschaften, insbesondere einen Kunststoff, oder einer gasgefüllten oder evakuierten Kammer.

Die spezifische Wärmeisolation verhindert den Wärmeübergang vom kälteren Medium B in das Ventilinnere, so dass es nicht zu einer Auskristallisation des im Medium A gelösten Feststoffes im Ventil kommt.

Das Ventil der vorliegenden Erfindung ist des Weiteren durch einen im Austrittsteil angeordneten Ventilkegel charakterisiert, wobei der Ventilkegel eine Vielzahl von Austrittsöffnungen mit einem Durchmesser von 1 bis 20 mm, bevorzugt 2 bis 10 mm, insbesondere bevorzugt 3 bis 5 mm aufweist.

Der Ventilkegel weist vorteilhafterweise 10 bis 1000, bevorzugt 100 bis 500, Austrittsöffnungen in Form von Bohrungen auf.

Der Ventilkegel ist also durch viele kleine Austrittsöffnungen perforiert. Dadurch kann die Anzahl und Größe der durch den Druckabfall und die Verdampfung der ersten Lösung entstehenden Gasblasen beeinflusst werden. Austrittsöffnungen mit einem kleineren Durchmesser sind bevorzugt, da diese die Bildung von kleinen, regelmäßigen Gasblasen bewirken, die das Auftreten von Dampfschlägen und damit mechanische Schwingungen in der Kreislaufleitung des Kristallisationsreaktors reduzieren bzw. verhindern. Es ist jedoch darauf zu achten, dass die Austrittsöffnungen nicht zu klein ausgebildet sind, da es hier zu Verstopfungen der Austrittsöffnungen kommen kann. Austrittsöffnungen mit einem zu großen Durchmesser verursachen auf der anderen Seite die Bildung von großen Blasen, und damit verbundenen Dampfschläge und Schwingungen mit hohen Amplituden. Je größer die Anzahl der Austrittsöffnungen des Ventilkegels ist, desto größer ist die Menge der ersten Lösung, die durch das Ventil strömt. Es ist somit auch vorteilhaft, die Regelmenge über die Anzahl der Austrittsöffnungen zu verändern und zu steuern.

Das Ventil ist vorteilhafterweise als Kolbenventil ausgebildet, welches durch die Austrittsöffnungen die Funktion eines Regelventils zur Mengenregelung erhält. Die Steuerung des Ventils erfolgt mittels eines üblichen Antriebs, insbesondere pneumatisch, elektrisch oder über einen Motor.

Mit Vorteil zirkuliert die zweite Lösung in einer Rohrleitung oder in einem Rührkessel und wird mit den üblich technischen Mitteln wie Umwälzpumpe oder Rührer in Bewegung bzw. Zirkulation gehalten.

Das Austrittsteil des Ventils ist dabei bevorzugt im Kern der Strömung, insbesondere parallel zur Strömungsrichtung, oder in einem spitzen Winkel zur Strömung der zweiten Lösung angeordnet.

Das erfindungsgemäße Ventil kommt in einer Kristallisationsanlage, insbesondere in einer Melaminkristallisationsanlage, zum Einsatz.

Die Aufgabe der vorliegenden Erfindung wird auch durch eine Kristallisationsanlage gemäß Anspruch 5 und einem Verfahren gemäß Anspruch 6 gelöst.

Die erfindungsgemäße Kristallisationsanlage zur kontinuierlichen Kristallisation eines gelösten Feststoffes, insbesondere Melamin, aus einer Lösung, weist mindestens einen Behälter für eine erste Lösung, mindestens eine Zirkulationseinheit für die zweite Lösung, und mindestens eine Verbindung zwischen dem Behälter mit der ersten Lösung und der Zirkulationseinheit mit der zweiten Lösung auf, wobei an der genannten Verbindung mindestens ein erfindungsgemäßes Ventil angeordnet ist

Es ist dabei von Vorteil, wenn der Zirkulationsstrom der zweiten Lösung größer ist als der Mengenstrom der eingespeisten Lösung, wobei das Verhältnis zwischen 10: 1 bis 100: 1, insbesondere zwischen 25: 1 bis 80: 1.

Das erfindungsgemäße Verfahren zur kontinuierlichen Kristallisation von gelösten Feststoffen, insbesondere Melamin, aus einem flüssigen Medium ist dadurch gekennzeichnet, dass eine erste Lösung mit einem gelösten Feststoff, insbesondere Melamin, in eine zweite zirkulierende Lösung über mindestens ein erfindungsgemäßes Ventil eingeführt bzw. eingedüst wird.

Es ist dabei für das Verfahren von Vorteil, wenn der Zirkulationsstrom der zweiten Lösung größer ist als der Mengenstrom der eingespeisten Lösung, wobei das Verhältnis zwischen 10: 1 bis 100: 1, insbesondere zwischen 25: 1 bis 80: 1.

Auch ist es für das Verfahren von Vorteil, wenn der Dampfdruck $p_{DTA}$, und die Temperatur $T_A$ der ersten Lösung größer sind als der Gesamtdruck $p_{gesB}$ und die Temperatur $T_B$ der zweiten Lösung.

So liegt die Temperatur $T_A$ der ersten Lösung vorteilhafterweise zwischen 100 bis 150°C, insbesondere zwischen 115 bis 145°C, und der Dampfdruck $p_{DTA}$ zwischen 2 bis 5 bar. Die zweite Lösung weist eine Temperatur $T_B$ zwischen 30 bis 50°C und einen Druck $p_{gesB}$ zwischen 0,5 und 2,5 bar auf.

Es ist ebenfalls vorteilhaft, wenn die Konzentration des Feststoffes in der ersten Lösung $C_A$ größer ist als die Konzentration des Feststoffes in der zweiten Lösung $C_B$ des in der zweiten gelösten Feststoffes.

Die Erfindung wird nachfolgend unter Bezugnahme auf die Figuren 1 bis 4 und mehreren Ausführungsbeispielen näher erläutert. Es zeigen:

Figur 1:     einen schematischen Aufbau eines in einer Melaminkristallisationsanlage verwendeten herkömmlichen Ventils

Figur 2     ein Diagramm mit Darstellung der in der Kristallisationsanlage auftretenden Dampfschläge bei Verwendung eines herkömmlichen Ventils

Figur 3     einen schematischen Aufbau des erfindungsgemäßen Ventils

Figur 4     ein Diagramm mit Darstellung der in der Kristallisationsanlage auftretenden Dampfschläge bei Verwendung eines erfindungsgemäßen Ventils

Figur 5     einen schematischen Querschnitt durch das erfindungsgemäße Ventil

**Ausführungsbeispiel 1:** Kristallisationsverfahren mit herkömmlichem Regelventil

Bei der Melaminkristallisation wird z.B. eine wässrige Melaminlösung 11 mit einer Temperatur von ca. 130° und einem Dampfdruck von ca. 2,5 bar in einen Kristallisationskreislaufstrom 12 von 40°C und einem Gesamtdruck von 1 bar eingespeist.

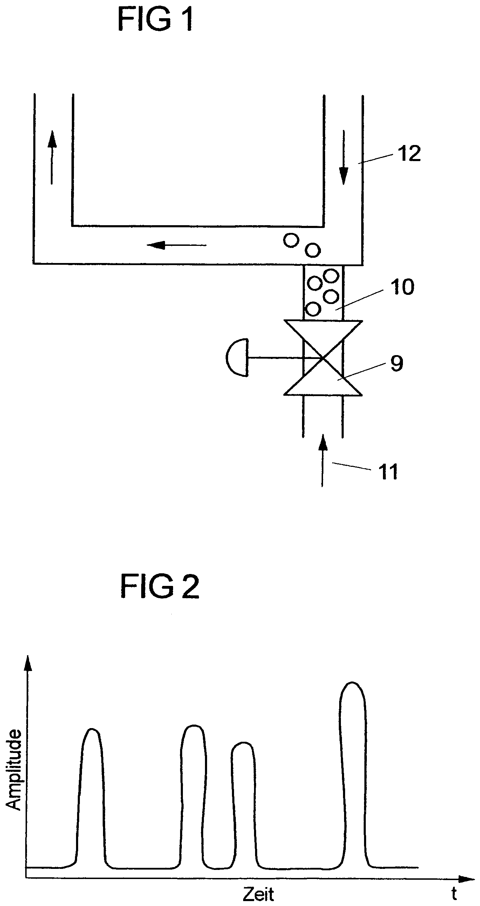

Bei Verwendung eines herkömmlichen Ventils 9 ist zwischen dem Punkt des Einleitens der wässrigen Melaminlösung 11 und dem Vermischen mit dem Kristallisationskreislaufstrom 12 ein Leitungszwischenstück 10 angeordnet (Figur 1).

In dem Zwischenstück 10 kommt es beim Einspeisen der heißen wässrigen Melaminlösung 11 in den Kristallisationskreislauf 12 zu einem sofortigen Druckabfall und der Bildung von großen Gasblasen ("Flash-Effekt"). Die Gasblasen treten in Kontakt mit dem kalten Kristallisationskreislauf 12, kondensieren und fallen dabei wieder zusammen. Dieser Vorgang verursacht heftige Schwingungen mit Amplituden bis zu 20 cm im Kristallisationssystem (Figur 2) und stellt eine erhebliche mechanische Belastung für die eingesetzten Bauteile dar sowie eine vorzeitige Kristallisation und Ausfällung des Melamins im Leitungszwischenstück 10 bzw. im Ventil.

**Ausführungsbeispiel 2:** Kristallisationsverfahren mit einem erfindungsgemäßen Ventil

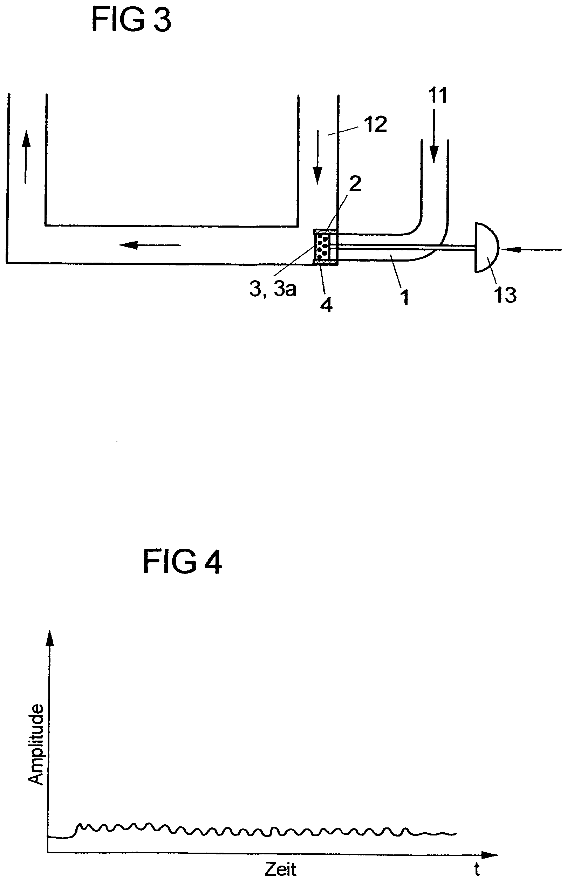

Bei Einsatz eines erfindungsgemäßen Ventils 1 erfolgt die Vermischung der wässrigen heißen Melaminlösung 11 mit dem kühleren Kristallisationskreislaufstrom 12 direkt im Kristallisationskreislauf und somit wird eine vorzeitige Melaminkristallisation und Ventilverstopfung verhindert (Figur 3).

Das Austrittsteil 2 des Ventils ist in Form eines Ventilkegels 3 ausgebildet und weist mehrere Austrittsöffnungen 3a mit einem Durchmesser von 4 mm auf. Das Ventil 1 wird durch den Antrieb 13 bewegt. Der Austrittsteil 2 ist im Kern der Strömung der zweiten Lösung 12 in Strömungsrichtung angeordnet.

Durch die Verwendung eines perforierten Ventilkegels 3, 3a werden eine Vielzahl von kleinen Blasen mit Durchmessern von ca. 4 mm gebildet, die im Gegensatz zu den großen Gasblasen des herkömmlichen Ventils 5 die mechanischen Schwingen der Anlage verhindern bzw. stark verringern. Wie aus dem Diagramm der Figur 4 ersichtlich, erfolgt eine sehr starke Reduzierung der Schwingungsamplitude.

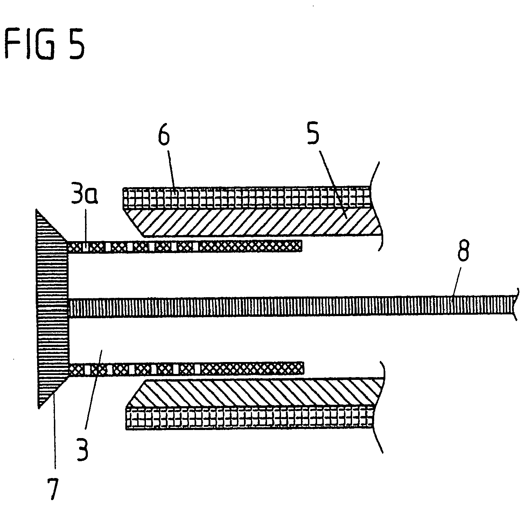

Figur 5 zeigt einen Ausschnitt aus einer weiteren bevorzugten Ausführungsform des erfindungsgemäßen Ventils 1. Das Ventil 1 weist ein Ventilgehäuse 5 auf, auf deren äußeren Fläche, die die Kontaktfläche 4 mit der zweiten Lösung darstellt, eine thermische Isolierung 6 angeordnet ist. Der Ventilkegel 3 weist mehrere Austrittsöffnungen 3a und auf der Frontseite des Kegels eine Dichtfläche 7 auf. Der Ventilkegel 3 wird mittels eines Antriebs über die Kegelstange 8 bewegt.

**Bezugszeichenliste**

1     erfindungsgemäßes Ventil
2     Austrittsteil des Ventils
3     Ventilkegel

3a     Austrittsöffnungen
4      Kontaktfläche des Austrittteils 2 mit der zweiten Lösung
5      Ventilgehäuse
6      thermische Isolierung
7      Dichtfläche
8      Kegelstange
9      herkömmliches Ventil
10    Leitungszwischenstück
11    heiße wässrige Melaminlösung als erste Lösung
12    Kristallisationskreislaufstrom als zweite Lösung
13    Antrieb

**Patentansprüche**

1. Ventil mit mindestens einem Austrittsteil zum Eindüsen einer feststoffhaltigen ersten Lösung in eine zweite Lösung zur kontinuierlichen Kristallisation des in der ersten Lösung gelösten Feststoffes in der zweiten Lösung **dadurch gekennzeichnet, dass** im Austrittsteil (2) mindestens ein Ventilkegel (3) angeordnet ist und der Ventilkegel (3) einen Kegelstumpf, einen zylindrischen Teil mit Austrittsöffnungen (3a) und eine Kegelstange (8) umfasst.

2. Ventil nach Anspruch 1, **dadurch gekennzeichnet, dass** mindestens eine Kontaktfläche (4) des Austrittsteils (2) mit der zweiten Lösung eine thermische Isolierung (6) aufweist, wobei die thermische Isolierung (6) aus einem Material mit schlechten Wärmeleiteigenschaften, insbesondere einen Kunststoff, oder einer gasgefüllten oder evakuierten Kammer besteht

3. Ventil nach Anspruch 1, **dadurch gekennzeichnet, dass** der Ventilkegel (3) 10 bis 1000, bevorzugt 100 bis 500 Austrittsöffnungen (3a) mit einem Durchmesser von 1 bis 20 mm, bevorzugt 2 bis 10 mm, insbesondere bevorzugt 3 bis 5 mm aufweist.

4. Verwendung eines Ventils nach einem der vorhergehenden Ansprüche in einer Kristallisationsanlage, insbesondere in einer Melaminkristallisationsanlage.

5. Kristallisationsanlage zur kontinuierlichen Kristallisation eines gelösten Feststoffes, insbesondere Melamin, aus einer Lösung, **gekennzeichnet durch** mindestens einen Behälter für eine erste Lösung, mindestens eine Zirkulationseinheit für eine zweite Lösung, mindestens eine Verbindung zwischen dem Behälter mit der ersten Lösung und der Zirkulationseinheit mit der zweiten Lösung, wobei an der genannten Verbindung mindestens einem Ventil nach einem der Ansprüche 1 bis 3 angeordnet ist.

6. Verfahren zur kontinuierlichen Kristallisation von gelösten Feststoffen, insbesondere Melamin, aus einem flüssigen Medium, **dadurch gekennzeichnet, dass** eine erste Lösung mit einem gelösten Feststoff, insbesondere Melamin, in eine zweite zirkulierende Lösung über mindestens ein Ventil nach einem der Ansprüche 1 bis 3 mit mindestens einem Austrittsteil eingeführt wird.

7. Verfahren nach Anspruch 6, **dadurch gekennzeichnet, dass** das mindestens eine Austrittsteil (2) des Ventils (1) im Betriebszustand in der Strömung der zweiten Lösung angeordnet ist und von dieser umspült wird,

8. Verfahren nach einem der Ansprüche 6 bis 7, **dadurch gekennzeichnet, dass** das Austrittsteil (2) des Ventils (1) im Kern der Strömung in Strömungsrichtung der zweiten Lösung angeordnet ist, wobei das Austrittsteil (2) des Ventils (1) parallel zur Strömung, insbesondere in Strömungsrichtung der zweiten Lösung, oder in einem spitzen Winkel zur Strömung der zweiten Lösung angeordnet ist.

9. Verfahren nach einem der Ansprüche 6 bis 8, **dadurch gekennzeichnet, dass** die zweite Lösung in einer Rohrleitung oder in einem Rührkessel zirkuliert.

10. Verfahren nach einem der Ansprüche 6 bis 9, **dadurch gekennzeichnet, dass** der Dampfdruck $p_{DTA}$, und die Temperatur $T_A$ der ersten Lösung größer sind als der Gesamtdruck $p_{gesB}$ und die Temperatur $T_B$ der zweiten Lösung.

**11.** Verfahren nach einem der Ansprüche 6 bis 10, **dadurch gekennzeichnet, dass** die erste Lösung eine Temperatur $T_A$ zwischen 100 bis 150°C, insbesondere zwischen 115 bis 145°C, und einen Dampfdruck $p_{DTA}$ zwischen 2 bis 5 bar aufweist.

**12.** Verfahren nach einem der Ansprüche 6 bis 11, **dadurch gekennzeichnet, dass** die zweite Lösung eine Temperatur $T_B$ zwischen 30 bis 50°C und einen Druck $p_{gesB}$ zwischen 0,5 und 2,5 bar aufweist.

**13.** Verfahren nach einem der Ansprüche 6 bis 12, **dadurch gekennzeichnet, dass** die Konzentration $C_A$ des in der ersten Lösung gelösten Feststoffes größer ist als die Konzentration $C_B$ des in der zweiten Lösung gelösten Feststoffes.

**14.** Verfahren nach einem der Ansprüche 6 bis 13, **dadurch gekennzeichnet, dass** der Zirkulationsstrom der zweiten Lösung größer ist als der Mengenstrom der eingespeisten ersten Lösung, wobei das Verhältnis zwischen 10: 1 bis 100: 1, insbesondere zwischen 25: 1 bis 80: 1 liegt.

**Claims**

**1.** Valve having at least one outlet part for injecting a first solution containing a solid material into a second solution for continuous crystallisation of the solid material, which is dissolved in the first solution, in the second solution, **characterised in that** at least one valve cone (3) is disposed in the outlet part (2), and the valve cone (3) includes a truncated cone, a cylindrical part with outlet openings (3a) and a conical rod (8).

**2.** Valve according to claim 1, **characterised in that** at least one contact surface (4) of the outlet part (2) with the second solution has a thermal insulation (6), the thermal insulation (6) consisting of a material with poor heat-conduction properties, in particular a plastic material, or a gas-filled or evacuated chamber.

**3.** Valve according to claim 1, **characterised in that** the valve cone (3) has 10 to 1,000, preferably 100 to 500, outlet openings (3a) with a diameter of 1 to 20 mm, preferably 2 to 10 mm, particularly preferably 3 to 5 mm.

**4.** Use of a valve according to one of the preceding claims in a crystallisation plant, in particular in a melamine crystallisation plant.

**5.** Crystallisation plant for continuous crystallisation of a dissolved solid material, in particular melamine, from a solution, **characterised by** at least one container for a first solution, at least one circulation unit for a second solution, at least one connection between the container with the first solution and the circulation unit with the second solution, at least one valve according to one of the claims 1 to 3 being disposed on the mentioned connection.

**6.** Method for continuous crystallisation of dissolved solid materials, in particular melamine, from a liquid medium, **characterised in that** a first solution with a dissolved solid material, in particular melamine, is introduced into a second circulating solution via at least one valve according to one of the claims 1 to 3 having at least one outlet part.

**7.** Method according to claim 6, **characterised in that** the at least one outlet part (2) of the valve (1) is disposed, in the operational state, in the flow of the second solution and is flushed by the latter.

**8.** Method according to one of the claims 6 to 7, **characterised in that** the outlet part (2) of the valve (1) is disposed in the core of the flow in flow direction of the second solution, the outlet part (2) of the valve (1) being disposed parallel to the flow, in particular in flow direction of the second solution, or at an acute angle to the flow of the second solution.

**9.** Method according to one of the claims 6 to 8, **characterised in that** the second solution circulates in a pipeline or in a stirred tank.

**10.** Method according to one of the claims 6 to 9, **characterised in that** the vapour pressure $p_{DTA}$ and the temperature $T_A$ of the first solution are greater than the total pressure $p_{gesB}$ and the temperature $T_B$ of the second solution.

**11.** Method according to one of the claims 6 to 10, **characterised in that** the first solution has a temperature $T_A$ between 100 to 150°C, in particular between 115 to 145°C, and a vapour pressure $p_{DTA}$ between 2 to 5 bar.

12. Method according to one of the claims 6 to 11, **characterised in that** the second solution has a temperature $T_B$ between 30 to 50°C and a pressure $p_{gesB}$ between 0.5 and 2.5 bar.

13. Method according to one of the claims 6 to 12, **characterised in that** the concentration $C_A$ of the solid material dissolved in the first solution is greater than the concentration $C_B$ of the solid material dissolved in the second solution.

14. Method according to one of the claims 6 to 13, **characterised in that** the circulation flow of the second solution is greater than the quantity flow of the supplied first solution, the ratio being between 10 : 1 to 100 : 1, in particular between 25 : 1 to 80 : 1.

## Revendications

1. Soupape ayant au moins une portion de sortie pour injecter dans une seconde solution une première solution contenant de la matière solide, afin de cristalliser en continu, dans la seconde solution, la matière solide dissoute dans la première solution,

   **caractérisée en ce que**
   dans la portion de sortie (2) est agencé au moins un cône de soupape (3) et le cône de soupape (3) comprend un tronc de cône, une partie cylindrique pourvue d'orifices de sortie (3a) et une tige de cône (8).

2. Soupape selon la revendication 1, **caractérisée en ce qu'**au moins une surface de contact (4) de la portion de sortie (2) avec la seconde solution comporte une isolation thermique (6), dans laquelle l'isolation thermique (6) est constituée d'un matériau ayant de mauvaises propriétés de conduction thermique, en particulier une matière plastique, ou d'une chambre remplie d'un gaz ou mise sous vide.

3. Soupape selon la revendication 1, **caractérisée en ce que** le cône de soupape (3) a de 10 à 1 000, de préférence de 100 à 500 orifices de sortie (3a) ayant un diamètre de 1 à 20 mm, de préférence de 2 à 10 mm, de manière plus préférée de 3 à 5 mm.

4. Utilisation d'une soupape selon l'une des revendications précédentes dans une installation de cristallisation, en particulier dans une installation de cristallisation de mélamine.

5. Installation de cristallisation destinée à cristalliser en continu une matière solide dissoute, en particulier de la mélamine, à partir d'une solution, **caractérisée par** au moins un réservoir pour une première solution, au moins une unité de circulation pour une seconde solution, au moins une liaison entre le réservoir contenant la première solution et l'unité de circulation contenant la seconde solution, dans laquelle au moins une soupape selon l'une des revendications 1 à 3 est agencée sur ladite liaison.

6. Procédé de cristallisation continue de matières solides dissoutes, en particulier de la mélamine, à partir d'un milieu liquide, **caractérisé en ce qu'**une première solution contenant des matières solides dissoutes, en particulier de la mélamine, est introduite dans une seconde solution en circulation par l'intermédiaire d'au moins une soupape selon l'une des revendications 1 à 3 comportant au moins une portion de sortie.

7. Procédé selon la revendication 6, **caractérisé en ce que** la au moins une portion de sortie (2) de la soupape (1) est agencée, en fonctionnement, dans l'écoulement de la seconde solution et est rincée par celle-ci.

8. Procédé selon l'une des revendications 6 à 7, **caractérisé en ce que** la portion de sortie (2) de la soupape (1) est agencée au coeur de l'écoulement dans la direction d'écoulement de la seconde solution, dans lequel fa portion de sortie (2) de la soupape (1) est agencée parallèlement à l'écoulement, en particulier dans la direction d'écoulement de la seconde solution, ou à un angle aigu par rapport à l'écoulement de la seconde solution.

9. Procédé selon l'une des revendications 6 à 8, **caractérisé en ce que** la seconde solution circule dans une conduite tubulaire ou dans une cuve de mélange.

10. Procédé selon l'une des revendications 6 à 9, **caractérisé en ce que** la pression de vapeur $p_{DTA}$ et la température

$T_A$ de la première solution sont supérieures à la pression totale $p_{gesB}$ et à la température $T_B$ de la seconde solution.

11. Procédé selon l'une des revendications 6 à 10, **caractérisé en ce que** la première solution a une température $T_A$ comprise entre 100 et 150 °C, en particulier entre 115 et 145 °C, et une pression de vapeur $p_{DTA}$ comprise entre 2 et 5 bar.

12. Procédé selon l'une des revendications 6 à 11, **caractérisé en ce que** la seconde solution a une température $T_B$ comprise entre 30 et 50 °C et une pression $p_{gesB}$ comprise entre 0,5 et 2,5 bar.

13. Procédé selon l'une des revendications 6 à 12, **caractérisé en ce que** la concentration $C_A$ de la matière solide dissoute dans la première solution est supérieure à la concentration $C_B$ de la matière solide dissoute dans la seconde solution.

14. Procédé selon l'une des revendications 6 à 13, **caractérisé en ce que** le débit circulant de la seconde solution est supérieur au débit massique de la première solution fournie, dans lequel le rapport est compris entre 10:1 et 100:1, en particulier entre 25:1 et 80:1.

# FIG 1

# FIG 2

# FIG 3

# FIG 4

FIG 5

EP 2 069 065 B1